# EUROPÄISCHE PATENTSCHRIFT

(11) **EP 1 453 784 B1**
(45) Veröffentlichungstag und Bekanntmachung des Hinweises auf die Patenterteilung: **12.09.2007**
(21) Anmeldenummer: 02804219.0
(22) Anmeldetag: 03.12.2002
(51) Int. Cl.: C07C 51/41, C07C 63/08, C07C 63/04, C07C 63/00

(54) **HERSTELLUNG VON AMMONIUMSALZEN AROMATISCHER CARBONSÄUREN**
PRODUCTION OF AMMONIUM SALTS OF AROMATIC CARBOXYLIC ACIDS
PRODUCTION DE SELS D'AMMONIUM D'ACIDES CARBOXYLIQUES AROMATIQUES

(30) Priorität: 04.12.2001 DE 10159420
(43) Veröffentlichungstag der Anmeldung: 08.09.2004
(73) Patentinhaber: BASF Aktiengesellschaft, 67056 Ludwigshafen (DE)
(72) Erfinder: KLOPP, Ingo, 67256 Weisenheim (DE); BOGENSTÄTTER, Thomas, 67098 Bad Dürkheim (DE); FRANKE, Dirk, 67134 Birkenheide (DE)
(74) Vertreter: Reitstötter - Kinzebach
(86) Internationale Anmeldenummer: PCT/EP2002/013678
(87) Internationale Veröffentlichungsnummer: WO 2003/048099

(56) Entgegenhaltungen:
- DE-B- 1 115 729
- US-A- 2 220 692
- US-A- 3 786 086
- DATABASE CA [Online] CHEMICAL ABSTRACTS SERVICE, COLUMBUS, OHIO, US; KOZLOV, S. K. ET AL: "Study of the ammonia-carboxylic acid interaction in an aprotic solvent" retrieved from STN Database accession no. 113:170597 XP002230602 in der Anmeldung erwähnt & ZHURNAL PRIKLADNOI KHIMII (SANKT-PETERBURG, RUSSIAN FEDERATION) (1990), 63(6), 1425-8 ,

## Beschreibung

Die vorliegende Erfindung betrifft ein Verfahren zur Herstellung von Ammoniumsalzen aromatischer Carbonsäuren, insbesondere der Benzoesäure.

Es ist bekannt, Ammoniumsalze aromatischer Carbonsäuren herzustellen, indem man gasförmigen Ammoniak in eine Lösung einer aromatischen Carbonsäure in einem aprotischen Lösungsmittel einleitet. Die DE 1115729 beschreibt ein derartiges Verfahren, wobei man als Lösungsmittel Dimethylformamid verwendet. Die US 2220692 beschreibt ein Verfahren zur Herstellung von Ammoniummandelat, bei dem eine Dispersion von Mandelsäure in wasserfreiem Benzol mit Ammoniak behandelt wird. Die DE 2005514 beschreibt ein Verfahren zur Herstellung von Thiolsulfinaten durch Oxidation der Episulfide mit einer organischen Persäure, insbesondere Perbenzoesäure, wobei die als Nebenprodukt anfallende Säure durch Einleiten von trockenem Ammoniak als Ammoniumsalz gefällt wird. Zur Fällung ist eine tiefe Temperatur von -50 bis -30 °C erforderlich.

Zh. Prikl. Khim. Bd.63, Nr. 6, S. 1425-1428 beschreibt ein Verfahren zur Herstellung von Ammoniumisobutyrat durch Umsetzung von Isobuttersäure mit Ammoniak in Isopentan. Der Einfluss des Ammoniakdrucks wird untersucht, allerdings lediglich im Hinblick auf die Anfangsbildungsgeschwindigkeit von Ammoniumisobutyrat.

Die bekannten Verfahren liefern zwar kristalline Ammoniumsalze, die Kristallgrößenverteilung ist jedoch breit und die Kristallgröße nicht einstellbar. Die erzielbaren Ausbeuten sind nicht befriedigend.

Der Erfindung liegt daher die Aufgabe zugrunde, ein Verfahren zur Herstellung von Ammoniumsalzen aromatischer Carbonsäuren anzugeben, das bei stöchiometrischem Einsatz der Reaktionspartner in im Wesentlichen quantitativer Ausbeute zu Kristallen definierter Größe mit enger Größenverteilung führt.

Erfindungsgemäß wird diese Aufgabe gelöst durch ein Verfahren zur Herstellung von Ammoniumsalzen aromatischer Carbonsäuren durch Umsetzung einer aromatischen Carbonsäure mit Ammoniak in einem aprotischen Lösungsmittel, das dadurch gekennzeichnet ist, dass man die Umsetzung in einem geschlossenen Gefäß durchführt, wobei man in das Gefäß kontinuierlich eine Lösung der aromatischen Carbonsäure in dem aprotischen Lösungsmittel einleitet, gasförmigen Ammoniak so einführt, dass man in dem Gasraum des Gefäßes einen Ammoniakpartialdruck im Bereich von 0,1 bis 3 bar aufrecht erhält, und eine Suspension des Ammoniumsalzes in dem aprotischen Lösungsmittel austrägt.

Als aromatische Carbonsäuren kommen solche Carbonsäuren mit wenigstens einem Benzolring in Betracht, bei denen die Carboxylgruppe entweder unmittelbar an den Benzolring oder über eine C₁-C₄-Alkylenkette daran geknüpft ist. Der Benzolring bzw. die Alkylenkette können unsubstituiert oder durch einen bis drei Substituenten, die unter C₁-C₄-Alkyl, Hydroxy, C₁-C₄-Alkoxy, Halogen oder Nitro ausgewählt sind, substituiert sein. Geeignete aromatische Carbonsäuren sind beispielsweise 2-Furancarbonsäure, 3-Furancarbonsäure, 2-Pyridincarbonsäure, 3-Pyridincarbonsäure und 4-Pyridincarbonsäure. Das erfindungsgemäße Verfahren eignet sich besonders zur Umsetzung von Benzoesäure.

Das erfindungsgemäß eingesetzte Lösungsmittel ist aprotisch (d. h. es weist kein acides Wasserstoffatom auf). Als aprotische Lösungsmittel kommen aliphatische Kohlenwasserstoffe, wie Pentan, Hexan, Heptan, Cyclohexan, Methylcyclohexan oder handelsübliche Gemische aliphatischer Kohlenwasserstoffe, wie bestimmte Raffinatfraktionen; aromatische Kohlenwasserstoffe, wie Benzol, Toluol, Xylol oder handelsübliche Gemische aromatischer Kohlenwasserstoffe, wie beispielsweise Solvesso®150; halogenierte aliphatische Kohlenwasserstoffe, wie Dichlormethan, Trichlormethan, 1,2-Dichlorethan, 1,2-Dichlorpropan, 1,1,2-Trichlorethan, Trichlorethylen; halogenierte aromatische Kohlenwasserstoffe, wie Chlorbenzol; Ether, wie Diethylether,Ethylenglykoldimethylether, Diethylenglykoldimethylether, Diisopropylether, Tetrahydrofuran, Dioxan; Ketone, wie Aceton, Methyl-tert-butylketon, Methylisobutylketon oder Methylethylketon; Dimethylformamid, Dimethylsulfoxid, Sulfolan und aliphatische oder aromatische Nitrile, wie Acetonitril oder Benzonitril in Betracht. Die zuvor genannten Lösungsmittel können einzeln oder in Form von Gemischen eingesetzt werden. Davon sind 1,2-Dichlorethan und 1,2-Dichlorpropan bevorzugt, wobei 1,2-Dichlorethan besonders bevorzugt ist.

Als Reaktionsgefäße für das erfindungsgemäße Verfahren sind übliche Reaktoren geeignet, die vorzugsweise rückvermischt sind, wie Rührreaktoren, Schlaufenreaktoren, Rührkesselkaskaden und dergleichen. Das Reaktionsgefäß ist vorzugsweise mit einem Rührorgan versehen, vorzugsweise einem solchen, dass eine gute Verteilung des Gases in der Flüssigkeit erlaubt, wie z. B. Scheibenrührer oder selbstansaugende Rührer. In das Reaktionsgefäß wird eine Lösung der aromatischen Carbonsäure in dem aprotischen Lösungsmittel eingeführt. Die Lösung kann oberhalb oder unterhalb der Flüssigkeitsoberfläche des im Gefäß vorliegenden Reaktionsgemisches eingeführt werden.

Gasförmiger Ammoniak wird so in das Reaktionsgefäß eingeführt, dass man in dem Gasraum des Gefäßes einen Ammoniakpartialdruck im Bereich von 0,1 bis 3 bar, vorzugsweise 0,1 bis 1 bar, aufrecht erhält. Zweckmäßigerweise bestimmt man den Ammoniakpartialdruck als Differenz des Gesamtdrucks im Reaktionsgefäß abzüglich des Systemdrucks, der im Wesentlichen vom Dampfdruck des Lösungsmittels bei der Reaktionstemperatur bestimmt wird. Der Systemdruck kann hinreichend genau ermittelt werden, indem man in das Reaktionsgefäß das gewählte Lösungsmittel einfüllt, das Reaktionsgefäß auf die Reaktionstemperatur erwärmt und den Druck im Reaktionsgefäß bestimmt, der dem Systemdruck entspricht. Wird nun Ammoniak in das Reaktionsgefäß eingebracht, so entspricht die Druckerhöhung gegenüber dem Systemdruck dem Ammoniakpartialdruck. Wird das erfindungsgemäße Verfahren bei konstanter Temperatur durchgeführt, so kann ein bestimmter Ammoniakpartialdruck aufrechterhalten werden, indem man den Gesamtdruck im Reaktionsgefäß überwacht und Ammoniak so zuführt, dass der Gesamtdruck konstant bleibt. Vorzugsweise erfolgen die Druckmessung und Regelung der Ammoniakzufuhr automatisiert. Der Gesamtdruck (Absolutdruck) beträgt vorzugsweise 1 bis 7 bar, insbesondere 1,1 bis 6 bar.

Der Ammoniak wird vorzugsweise unterhalb der Flüssigkeitsoberfläche in das Gefäß eingeführt. Vorzugsweise zieht man kontinuierlich Gas aus dem Gasraum des Gefäßes ab, versetzt dieses mit frischem Ammoniak und führt es in das Gefäß zurück. Diese Vorgehensweise gestattet einen optimalen Ammoniak-Umsatz.

Aus dem Reaktionsgefäß wird, vorzugsweise am Boden, eine Suspension des Ammoniumsalzes ausgetragen. Der Abzug der Suspension erfolgt vorzugsweise kontinuierlich und vorzugsweise so, dass die Flüssigkeits-Füllhöhe oder die Flüssigkeitsmenge im Gefäß konstant bleibt. Die entnommene Suspension kann direkt oder nach Passage durch einen Wärmetauscher weiteren Reaktionsstufen zugeführt werden. Der Restgehalt an aromatischer Carbonsäure, der z. B. mittels Hochdruckflüssigkeitschromatographie (HPLC) bestimmt werden kann, beträgt typischerweise weniger als 1000 ppm, meist weniger als 200 ppm. Gewünschtenfalls kann das Ammoniumsalz nach üblichen Trennverfahren, z. B. durch Filtration oder Zentrifugation vom Lösungsmittel abgetrennt werden. Das abgetrennte Lösungsmittel kann in das Verfahren zurückgeführt werden.

Das erfindungsgemäße Verfahren weist allgemein eine geringe Konzentration an Ammoniak und aromatischer Carbonsäure in der flüssigen Phase und somit geringe Keimbildungsraten auf. Dies bedingt, dass die aromatische Carbonsäure und der Ammoniak in Gegenwart von vorgebildeten Kristallen des Ammoniumsalzes zur Reaktion gelangen, die als Impfkristalle dienen. Daher beruht das erfindungsgemäße Verfahren - im Unterschied zu den Verfahren des Standes der Technik - nicht auf einer spontanen Kristallisation, die üblicherweise zu sehr breiten Kristallgrößenverteilungen führt.

Die Temperatur im Reaktionsgefäß wird vorzugsweise konstant gehalten. Die optimale Reaktionstemperatur bemisst sich nach der Löslichkeit des Ammoniumsalzes in dem verwendeten Lösungsmittel. Hohe Temperaturen erhöhen die Löslichkeit des Ammoniumsalzes und fördern damit das Kristallwachstum, führen aber auch zu stärkerer Agglomeratbildung und beim Abkühlen zur Ausfällung von feinkristallinem Material. Bei tiefen Temperaturen ist die Löslichkeit des Ammoniumsalzes zu gering, so dass hohe Übersättigungen und demzufolge nur sehr feine Kristalle gebildet werden. Vorzugsweise wird die Temperatur so gewählt, dass die Löslichkeit der aromatischen Carbonsäure in dem Lösungsmittel mehr als 10 g/100 ml, insbesondere mehr als 35 g/100 ml, und die Löslichkeit des Ammoniumsalzes vorzugsweise weniger als 2 g/100 ml, insbesondere weniger als 1 g/100 ml, beträgt. Eine Temperatur im Bereich von 70 bis 110 °C, vorzugsweise 80 bis 95°C, hat sich beispielsweise bei Verwendung von 1,2-Dichlorethan als Lösungsmittel als geeignet erwiesen.

Die mittlere Verweilzeit im Reaktionsgefäß beträgt vorzugsweise 10 bis 300 min. Die untere Grenze der mittleren Verweilzeit ist durch den Stoffübergang zwischen Gas und Flüssigkeit limitiert, eine längere Verweilzeit macht das Verfahren unwirtschaftlich und führt zu breiteren Kristallgrößenverteilungen.

Das erfindungsgemäße Verfahren gestattet einen praktisch quantitativen Umsatz der eingesetzten aromatischen Carbonsäure. Die Kristalle des Ammoniumsalzes fallen als Suspension in einem Lösungsmittel an, das von gelöster aromatischer Carbonsäure praktisch frei ist. Die Suspension kann ohne Filtration oder Eindampfung in Folgereaktionen eingesetzt werden. Das Verfahren gestattet die Einstellung definierter Kristallgrößen mit enger Größenverteilung.

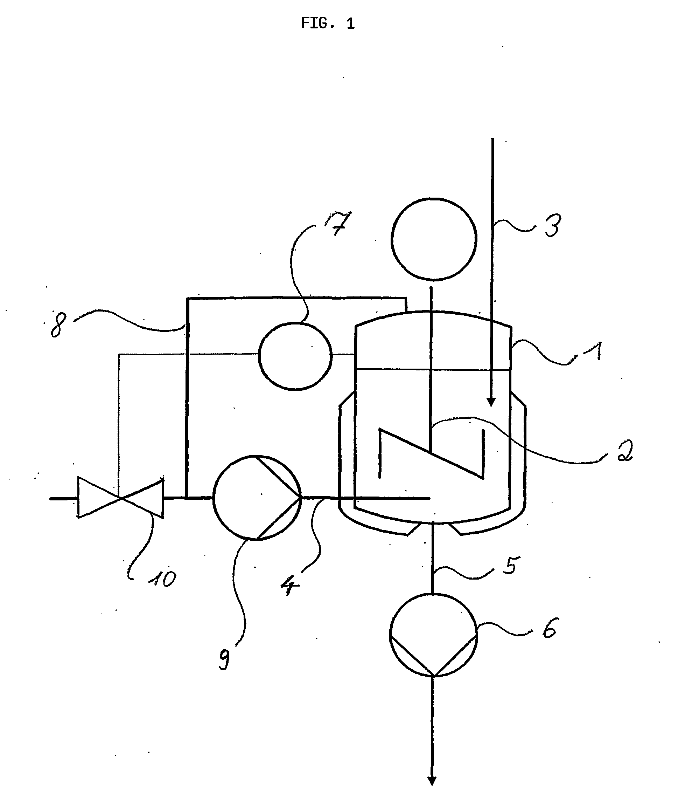

Die Erfindung wird durch die beigefügte Figur 1 und die nachfolgenden Beispiele näher veranschaulicht. Die in den Beispielen erhaltenen Kristallisate wurden unter dem Lichtmikroskop begutachtet; die Teilchengrößenverteilung wurde nach der Methode der Laserextinktionsmessung mittels eines Partikelzählers bestimmt (Meßbereich 2-400 µm; Sensor 400 µm x 400 µm). Es ist jeweils der Maximalwert und die Spannweite der Verteilung (Spannweite = (X₉₀-X₁₀/X₅₀)) angegeben. Zur Bestimmung nicht umgesetzter Benzoesäure ließ man die Suspension erkalten, filtrierte das Ammoniumbenzoat ab und analysierte das Filtrat durch Hochdruckflüssigkeitschromatographie.

Figur 1 zeigt eine zur Durchführung des erfindungsgemäßen Verfahrens geeignete Anlage. In den Reaktor 1, der mit einem Rührorgan 2 versehen ist, wird über die Leitung 3 eine Lösung einer aromatischen Carbonsäure in einem aprotischen Lösungsmittel eingeführt. Über die Leitung 4 wird unterhalb der Flüssigkeitsoberfläche gasförmiger Ammoniak eingeperlt. Am Boden des Reaktors 1 wird über die Leitung 5 und die Pumpe 6 eine Suspension des gebildeten Ammoniumsalzes abgepumpt. Aus dem Gasraum des Reaktors 1 wird über die Leitung 8 und die Pumpe 9 Gas abgezogen, über das Dosierventil 10 mit frischem Ammoniak versetzt und über Leitung 4 in den Reaktor 1 zurückgeführt. Das Dosierventil 10 steht unter der Kontrolle des Druckreglers 7.

### Beispiel 1

In einem 2 L Doppelmantelreaktor, der mit einem Scheibenrührer und Stromstörern ausgerüstet war, wurden gleichzeitig getaucht 53,3 g min⁻¹ einer Lösung von 720 g Benzoesäure in 3280 g 1,2-Dichlorethan (DCE) und gasförmiger Ammoniak zugefahren. Die Reaktionstemperatur betrug 90 °C, der Gesamtdruck 1,6 bar und der Ammoniakpartialdruck 0,38 bar. Aus dem Gasraum wurden über eine Pumpe mit 50 l h⁻¹ Gas umgepumpt und getaucht zugeführt. Die Verweilzeit im Reaktor wurde auf 45 Minuten eingestellt. Die entstehende Ammoniumbenzoat-Suspension wurde über ein Bodenventil kontinuierlich ausgefördert. Nach 8 Verweilzeiten wurde eine Probe analysiert: grob kristallines, nicht agglomeriertes Ammoniumbenzoat einer mittleren Teilchengröße von ca. 300 µm (die Teilchengrößenverteilung konnte nicht bestimmt werden, da die Teilchen den Sensor des Partikelzählers zusetzten); gelöste Benzoesäure: < 10 ppm.

### Beispiel 2

Dem im Beispiel 1 beschriebenen Reaktor wurden gleichzeitig getaucht 53,3 g min⁻¹ einer Lösung von 720 g Benzoesäure in 3280 g DCE und gasförmiger Ammoniak zugeführt. Die Reaktionstemperatur wurde auf 80 bis 81 °C, der Gesamtdruck auf 1,3 bar und der Ammoniakpartialdruck auf 0,37 bar gehalten. Nicht umgesetzter Ammoniak wurde über eine Pumpe mit 60 l h⁻¹ als Kreisgas umgewälzt. Die Verweilzeit im Reaktor wurde auf 45 Minuten eingestellt. Die entstehende Ammoniumbenzoat-Suspension wurde über das Bodenventil kontinuierlich ausgefördert. Nach 8 Verweilzeiten wurde eine Probe analysiert: grob kristallines, nicht agglomeriertes Ammoniumbenzoat; gelöste Benzoesäure: < 5 ppm; Teilchengrößenverteilung: Maximum 90 µm, Spannweite 0,93.

### Beispiel 3:

Dem im Beispiel 1 beschriebenen Reaktor wurden gleichzeitig getaucht 53,3 g min⁻¹ einer Lösung von 720 Benzoesäure in 3280 g DCE und gasförmiger Ammoniak zugeführt. Die Reaktionstemperatur wurde auf 81 °C, der Gesamtdruck auf 2,1 bar und der Ammoniakpartialdruck auf 1,19 bar eingestellt. Nicht umgesetzter Ammoniak wurde über eine Pumpe mit 60 l h⁻¹ als Kreisgas umgewälzt. Die Verweilzeit im Reaktor wurde auf 45 Minuten eingestellt. Die entstehende Ammoniumbenzoat-Suspension wurde über das Bodenventil kontinuierlich ausgefördert. Nach 8 Verweilzeiten wurde eine Probe analysiert: fein kristallines, agglomeriertes Ammoniumbenzoat; gelöste Benzoesäure: 5 ppm; Teilchengrößenverteilung: Maximum 60 µm, Spannweite 0,92.

### Vergleichsbeispiel 1:

Dem im Beispiel 1 beschriebenen Reaktor wurden gleichzeitig getaucht 53,3 g min⁻¹ einer Lösung von 720 g Benzoesäure in 3280 g DCE und 83,0 g h⁻¹ gasförmiger Ammoniak zugeführt. Die Reaktionstemperatur wurde auf 80 °C, der Gesamtdruck auf 1,0 bar eingestellt. Der Ammoniakpartialdruck betrug 0,07 bar. Nicht umgesetzter Ammoniak wurde über eine Pumpe mit 50 l h⁻¹ als Kreisgas umgewälzt. Die Verweilzeit im Reaktor wurde auf 45 Minuten eingestellt. Die entstehende Ammoniumbenzoat-Suspension wurde über das Bodenventil kontinuierlich ausgefördert. Nach 8 Verweilzeiten wurde eine Probe analysiert: fein kristallines, stark agglomeriertes Ammoniumbenzoat; gelöste Benzoesäure: 1800 ppm; Teilchengrößenverteilung: Maximum 290 µm, Spannweite 2,2.

### Vergleichsbeispiel 2:

Einem 2 L Planschliffreaktor mit Rückflußkühler, Rührer und Stromstörer wurden gleichzeitig getaucht 53.3 g min⁻¹ einer Lösung von 720 g Benzoesäure in 3280 g 1,2-Dichlorethan und 1.4 g min⁻¹ gasförmiger Ammoniak zugeführt. Die Reaktion wurde drucklos gefahren. Die Temperatur im Reaktor betrug 80 - 82 °C. Nicht umgesetzter Ammoniak konnte über den Rückflußkühler entweichen. Die Ammoniumbenzoatsuspension wurde kontinuierlich über ein Bodenventil ausgefördert. Die Verweilzeit wurde auf 45 min eingestellt. Nach 6 Verweilzeiten wurde eine Probe entnommen und analysiert: Gemisch aus fein kristallinem, stark agglomeriertem Ammoniumbenzoat mit größeren Kristallen; gelöste Benzoesäure: 7200 ppm: Teilchengrößenverteilung: Maximum 67 µm, Spannweite 1,24.

### Vergleichsbeispiel 3:

In dem im Vergleichsbeispiel 2 beschriebenen Reaktor wurden 600 g 1,2-Dichlorethan vorgelegt und zum Rückfluss erhitzt (etwa 84 °C). Dann wurden gleichzeitig innerhalb von 45 Minuten eine Lösung von 558,2 g Benzoesäure in 2400 g 1,2-Dichlorethan und 85,6 g gasförmiger NH₃ zugeführt. Die Reaktionstemperatur lag zwischen 81-82 °C. Die Reaktion wurde drucklos gefahren. Nicht umgesetzter NH₃ entwich über den Rückflusskühler. Nach Ende der Zudosierungen wurde eine Probe aus dem Reaktor entnommen und analysiert: Gemisch aus fein kristallinem, stark agglomeriertem Ammoniumbenzoat, Teilchengröße < 15 µm und gröberen Kristallen von etwa 50 µm; gelöste Benzoesäure: 50 ppm; Teilchengrößenverteilung: Maximum 20 µm, Spannweite 1,53 (Verteilung war bimodal).

## Patentansprüche

1. Verfahren zur Herstellung von Ammoniumsalzen aromatischer Carbonsäuren durch Umsetzung einer aromatischen Carbonsäure mit Ammoniak in einem aprotischen Lösungsmittel, **dadurch gekennzeichnet, dass** man die Umsetzung in einem geschlossenen Gefäß durchführt, wobei man in das Gefäß kontinuierlich eine Lösung der aromatischen Carbonsäure in dem aprotischen Lösungsmittel einleitet und gasförmigen Ammoniak so einführt, dass man in dem Gasraum des Gefäßes einen Ammoniakpartialdruck im Bereich von 0,1 bis 3 bar aufrecht erhält, und eine Suspension des Ammoniumsalzes in dem aprotischen Lösungsmittel austrägt.

2. Verfahren nach Anspruch 1, **dadurch gekennzeichnet, dass** man den Ammoniak unterhalb der Flüssigkeitsoberfläche des Reaktionsgemisches in das Gefäß einführt.

3. Verfahren nach Anspruch 2, **dadurch gekennzeichnet, dass** man kontinuierlich Gas aus dem Gasraum des Gefäßes abzieht, mit frischem Ammoniak versetzt und in das Gefäß zurückführt.

4. Verfahren nach einem der vorhergehenden Ansprüche, **dadurch gekennzeichnet, dass** das aprotische Lösungsmittel ausgewählt ist unter aliphatischen Kohlenwasserstoffen, aromatischen Kohlenwasserstoffen, halogenierten aliphatischen Kohlenwasserstoffen, halogenierten aromatischen Kohlenwasserstoffen, Ethern, Ketonen, Dimethylformamid, Dimethylsulfoxid, Sulfolan, aliphatischen oder aromatischen Nitrilen und Gemischen davon.

5. Verfahren nach Anspruch 4, **dadurch gekennzeichnet, dass** man als aprotisches Lösungsmittel 1,2-Dichlorethan oder 1,2-Dichlorpropan verwendet.

6. Verfahren nach einem der vorhergehenden Ansprüche, **dadurch gekennzeichnet, dass** man in dem Gefäß eine Temperatur von 70 bis 110 °C einhält.

7. Verfahren nach einem der vorhergehenden Ansprüche, **dadurch gekennzeichnet, dass** man in dem Gefäß einen Gesamtdruck von 1 bis 7 bar einstellt.

8. Verfahren nach einem der vorhergehenden Ansprüche, **dadurch gekennzeichnet, dass** die mittlere Verweilzeit im Gefäß 10 bis 300 min beträgt.

9. Verfahren nach einem der vorhergehenden Ansprüche, **dadurch gekennzeichnet, dass** man als aromatische Carbonsäure Benzoesäure verwendet.

## Claims

1. A process for preparing ammonium salts of aromatic carboxylic acids by reacting an aromatic carboxylic acid with ammonia in an aprotic solvent, which comprises carrying out the reaction in a closed vessel by continuously introducing a solution of the aromatic carboxylic acid in the aprotic solvent and passing in gaseous ammonia so that an ammonia partial pressure in the range from 0.1 to 3 bar is maintained in the gas space of the vessel, and discharging a suspension of the ammonium salt in the aprotic solvent.

2. The process according to claim 1, wherein the ammonia is passed into the vessel below the liquid surface of the reaction mixture.

3. The process according to claim 2, wherein gas is continuously withdrawn from the gas space of the vessel, admixed with fresh ammonia and passed back into the vessel.

4. The process according to any of the preceding claims, wherein the aprotic solvent is selected from aliphatic hydrocarbons, aromatic hydrocarbons, halogenated aliphatic hydrocarbons, halogenated aromatic hydrocarbons, ethers, ketones, dimethylformamide, dimethyl sulfoxide, sulfolane, aliphatic or aromatic nitriles and mixtures thereof.

5. The process according to claim 4, wherein the aprotic solvent is 1,2-dichloroethane or 1,2-dichloropropane.

6. The process according to any of the preceding claims, wherein a temperature of from 70 to 110°C is maintained in the vessel.

7. The process according to any of the preceding claims, wherein an overall pressure of from 1 to 7 bar is maintained in the vessel.

8. The process according to any of the preceding claims, wherein the average residence time in the vessel is from 10 to 300 min.

9. The process according to any of the preceding claims, wherein the aromatic carboxylic acid is benzoic acid.

## Revendications

1. Procédé de préparation de sels d'ammonium d'acides carboxyliques aromatiques par réaction d'un acide carboxylique aromatique avec de l'ammoniac dans un solvant aprotique, **caractérisé en ce que** la réaction est mise en oeuvre dans un récipient fermé, une solution de l'acide carboxylique aromatique dans le solvant aprotique étant introduite de manière continue dans le récipient et de l'ammoniac gazeux étant introduit de manière à ce qu'une pression partielle d'ammoniac dans la plage de 0,1 à 3 bars soit maintenue dans l'espace gazeux du récipient, et qu'il en résulte une suspension du sel d'ammonium dans le solvant aprotique.

2. Procédé selon la revendication 1, **caractérisé en ce que** l'ammoniac est introduit dans le récipient au-dessous de la surface de liquide du mélange réactionnel.

3. Procédé selon la revendication 2, **caractérisé en ce que** du gaz est retiré de manière continue de l'espace gazeux du récipient, melangé à de l'ammoniac frais et reconduit dans le récipient.

4. Procédé selon l'une quelconque des revendications précédentes, **caractérisé en ce que** le solvant aprotique est sélectionné parmi des hydrocarbures aliphatiques, des hydrocarbures aromatiques, des hydrocarbures aliphatiques halogénés, des hydrocarbures aromatiques halogénés, des éthers, des cétones, du diméthylformamide, du diméthylsulfoxyde, du sulfolane, des nitriles aliphatiques ou aromatiques et des mélanges de ceux-ci.

5. Procédé selon la revendication 4, **caractérisé en ce que** du 1,2-dichloroéthane ou du 1,2-dichloropropane est utilisé en tant que solvant aprotique.

6. Procédé selon l'une quelconque des revendications précédentes, **caractérisé en ce qu'**une température de 70 à 110°C est maintenue dans le récipient.

7. Procédé selon l'une quelconque des revendications précédentes, **caractérisé en ce qu'**une pression totale de 1 à 7 bars est ajustée dans le récipient.

8. Procédé selon l'une quelconque des revendications précédentes, **caractérisé en ce que** le temps de séjour moyen dans le récipient est de 10 à 300 minutes.

9. Procédé selon l'une quelconque des revendications précédentes, **caractérisé en ce que** de l'acide benzoïque est utilisé en tant qu'acide carboxylique aromatique.
